# EUROPÄISCHE PATENTANMELDUNG

(11) **EP 2 279 689 A1**
(43) Veröffentlichungstag der Anmeldung: **02.02.2011**
(21) Anmeldenummer: 09166857.4
(22) Anmeldetag: 30.07.2009
(51) Int. Cl.: A61B 5/00, A61B 5/15

(54) **Medizinisches Handgerät**

(71) Anmelder: F. Hoffmann-La Roche AG, 4070 Basel (CH); Roche Diagnostics GmbH, 68305 Mannheim (DE)
(72) Erfinder: Eisenhardt, Christoph, 68165 Mannheim (DE); Haupt, Martin, 1230 Wien (AT)
(74) Vertreter: Pfiz, Thomas

(57) **Zusammenfassung**

Die Erfindung betrifft ein Medizinisches Handgerät mit einer in ein Gerätegehäuse (26) einsetzbaren Testbandeinheit (14), die ein mit einer Vielzahl von analytischen Testelementen (18) versehenes, mittels einer Spule (22) aufwickelbares Testband (16) umfasst, und einem mit der Testbandeinheit (14) koppelbaren, einen Elektromotor (34) und ein Getriebe (36) umfassenden Bandantrieb (12) zum abschnittsweisen Vorspulen des Testbands (16), so dass die Testelemente (18) zur Probenapplikation sukzessive bereitstellbar sind. Erfindungsgemäß wird vorgeschlagen, dass die Drehachse des Elektromotors (34) in einer quer zur Drehachse der Spule (22) sich erstreckenden Ebene angeordnet ist, und dass das Getriebe (36) einen Winkeltrieb (58) zur Achsumlenkung aufweist.

## Beschreibung

Die Erfindung betrifft ein medizinisches Handgerät, insbesondere für Blutzuckertests, mit einer in ein Gerätegehäuse einsetzbaren Testbandeinheit, die ein mit einer Vielzahl von analytischen Testelementen versehenes, auf einer Spule aufwickelbares Testband umfasst, und einem mit der Testbandeinheit koppelbaren, einen Elektromotor und ein Getriebe umfassenden Bandantrieb zum abschnittsweisen Vorspulen des Testbands, so dass die Testelemente zur Probenapplikation sukzessive bereitstellbar sind.

Derartige Testbandinstrumente sollen gegenüber den am Markt befindlichen Streifensystemen weitere Anwendervorteile gewähren. Für die Praxis muss neben einer zuverlässigen Positionierung der Testelemente auch eine diskrete Gerätebedienung gewährleistet sein. So dürfen bei der Benutzung keine übermäßigen Betriebsgeräusche auftreten, die durch Personen in der Umgebung wahrgenommen werden. Um die Herstellkosten beispielsweise für ein Blutzuckermessgerät in akzeptablem Rahmen zu halten, müssen in der Regel preisgünstige Komponenten verwendet werden, bei denen die Einhaltung kleiner Toleranzen problematisch ist. Als weitere Anforderungen sollte das Bauvolumen reduziert sein und die Energieausbeute des Antriebsstrangs möglichst hoch sein. Ausgehend hiervon liegt der Erfindung die Aufgabe zugrunde, die im Stand der Technik bekannten Systeme weiter zu verbessern und eine sichere Testelementpositionierung mit geringen Störgeräuschen bei kompaktem Aufbau zu erreichen.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Anordnung des Antriebsstranges speziell für den Bandtransport zu optimieren. Dementsprechend wird erfindungsgemäß vorgeschlagen, dass die Drehachse des Elektromotors in einer quer zur Drehachse der Spule sich erstreckenden Ebene angeordnet ist, und dass das Getriebe einen Winkeltrieb zur Achsumlenkung zwischen der Motorachse und der Spulenachse aufweist. Damit ist es möglich, eine Drehbewegung über winklig zueinander stehende Achsen zu übertragen. Dies ermöglicht eine an die Besonderheit des Bandtransports angepasste sehr geringe Bauhöhe, während zugleich auf kostengünstigere Antriebe in Form von konventionellen Kleinmotoren zurückgegriffen werden kann, ohne dass allzu hohe Anforderungen an die mechanische Präzision entstehen.

Eine besonders bevorzugte Ausführung sieht vor, dass der Winkeltrieb durch eine Kronenradgetriebestufe gebildet ist. Die Kraftübertragung erfolgt hierbei unter Zahneingriff, wobei im Vergleich zu einem Schneckengetriebe ein wesentlich höherer Wirkungsgrad erzielt wird und zudem eine hohe Drehzahlreduktion in einer Getriebestufe möglich ist.

Vorteilhafterweise besitzt die Kronenradgetriebestufe ein Kronenrad und ein zylindrisches Zahnrad mit Geradverzahnung. Damit wird im Gegensatz zu Kegelrädern die axiale Toleranz weitgehend unkritisch, dass das geradverzahnte Zahnrad entlang seiner Achse verschoben werden kann, ohne dass sich Probleme beim Laufverhalten ergeben. Außerdem entstehen auch unter Last keine axialen Kräfte.

Eine weitere besonders vorteilhafte Ausführung sieht vor, dass das Getriebe eine Hohlradgetriebestufe aufweist, wobei die Hohlradgetriebestufe durch ein innenverzahntes Hohlrad und ein mit seiner Achse innerhalb des Hohlrads liegendes, auf der Motorwelle des Elektromotors sitzendes Antriebsritzel gebildet ist. Dadurch lässt sich die Drehzahl schon im ersten Schritt erheblich reduzieren, so dass störende Geräusche effektiv vermindert werden.

Eine weitere Verbesserung in dieser Hinsicht ergibt sich dadurch, dass die Hohlradgetriebestufe zur Drehzahlreduzierung ein Übersetzungsverhältnis von mehr als 2, vorzugsweise etwa 4 aufweist.

Vorteilhafterweise weist das Hohlrad konkave Zahnflanken und das Antriebsritzel konvex gewölbte Zahnflanken auf. Dies führt zu einer größeren Zahnüberlappung und damit auch zu einem "weicheren" Geräusch.

Von Vorteil ist es auch, wenn die mit dem Elektromotor direkt gekoppelte Hohlradgetriebestufe an einer Lagerstelle an dem Elektromotor gelagert ist. Damit ist es möglich, Toleranzen beim wichtigen Abstand zwischen Motorwelle/Ritzel und Innenverzahnung der ersten Stufe kostengünstig zu minimieren. In diesem Zusammenhang ist es auch günstig, wenn das Antriebsritzel und ggf. die Lagerhalterung für das Hohlrad als metallische Bauteile ausgebildet sind.

Eine besonders günstige Drehzahlanpassung bei reduziertem Geräuschverhalten ergibt sich dadurch, dass die Kronenradgetriebestufe der Hohlradgetriebestufe nachgeordnet ist, wobei die Motordrehzahl des Elektromotors ein Mehrfaches der Eingangsdrehzahl der Kronenradgetriebestufe beträgt.

Um die Antriebskräfte zuverlässig auf die Spule übertragen zu können, ist es von Vorteil, eine mehrere Stirnzahnräder umfassende, abtriebseitig an einen Antriebszapfen direkt mit der Spule koppelbare Stirnradgetriebestufe einzusetzen.

Von Vorteil hinsichtlich effizienter Bewegungsübertragung ist es auch, wenn die Stirnzahnräder gemeinsam auf einer Seite einer Lagerplatte gelagert sind, wobei das ausgangsseitige Stirnzahnrad über einen Spulenteller die Spule direkt antreibt.

Um eine preisgünstige und zugleich kompakte Ausführung zu realisieren, ist es vorteilhaft, wenn der Elektromotor durch einen aus einer geräteseitigen Energieversorgung gespeisten Gleichstrom-Kleinmotor mit einer Drehzahl im Bereich von 5000 bis 15000, vorzugsweise etwa 9000 Umdrehungen pro Minute gebildet ist.

Ein wesentlicher Vorteil liegt neben der Geräuschminimierung auch in der Verwirklichung einer kompakten Bauweise. Hierfür ist es besonders günstig, wenn der Elektromotor einen langgestreckten Motorblock aufweist, und dass die Motorwelle in Längsrichtung des Motorblocks verläuft. Um ein axiales Spiel weiter zu verringern, ist es vorteilhaft, wenn die Motorwelle des Elektromotors in einem aus Sintermaterial bestehenden Sinterlager gelagert ist.

Um die Gebrauchsvorteile weiter zu verbessern, ist das Gerätegehäuse zum Einwechseln einer Bandkassette als Testbandeinheit ausgebildet, wobei die maximale Dicke des flachen Gerätegehäuses weniger als 30mm, vorzugsweise weniger als 25mm beträgt.

Im Folgenden wird die Erfindung anhand eines in der Zeichnung dargestellten Ausführungsbeispiels näher erläutert. Es zeigen:
- Fig. 1: ein medizinisches Handgerät mit einer Band- kassette für Blutzuckertests in einer aufge- brochenen perspektivischen Darstellung;
- Fig. 2: den Bandantrieb des Handgeräts in einer ge- genüber Fig. 1 gedrehten Vergrößerung;
- Fig. 3: den Bandantrieb umfassend einen Elektromotor und ein Zahnradgetriebe in einer Seitenan- sicht;
- Fig. 4: zwei Getriebestufen des Bandantriebs in per- spektivischer Darstellung;
- Fig. 5: eine ausschnittsweise Vergrößerung der Hohl- radgetriebestufe nach Fig. 4; und
- Fig. 6: ein Diagramm des Drehzahlverlaufs über den Getriebestrang.

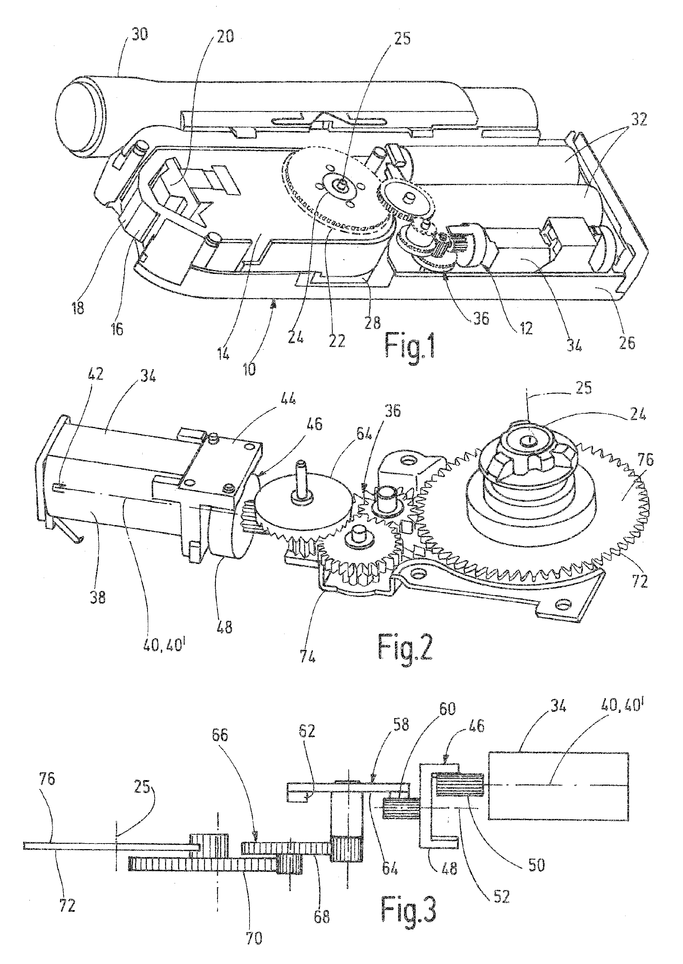

Das in Fig. 1 dargestellte Analysehandgerät umfasst eine Geräteeinheit 10 mit einem besonders laufruhig ausgestalteten Bandantrieb 12 und eine Testbandeinheit in Form einer wechselbaren Bandkassette 14, die mit dem Bandantrieb koppelbar ist. Mit dem Gerät lassen sich Blutzuckermessungen vor Ort vom Anwender selbst vornehmen.

Die in Fig. 1 von der Unterseite gezeigte Bandkassette 14 enthält ein Testband 16, das abschnittsweise mit Testelementen bzw. Testfeldern 18 versehen ist, die im Bereich einer Umlenkspitze als Applikationsstelle an ihrer freien Vorderseite mit Blut bzw. Körperflüssigkeit beaufschlagbar sind. Zugleich kann dort eine rückseitige Vermessung einer durch einen Analyten (Blutglucose) bedingten Farbänderung des jeweiligen Testfelds 18 durch die optische Messeinheit 20 erfolgen. Das Testband 16 wird zu diesem Zweck mittels des Bandantriebs 12 aus einem Vorrat der Kassette 14 abgezogen und auf eine Aufwickelspule 22 umgespult, so dass die voneinander beabstandeten Testfelder 18 nacheinander an der Applikationsstelle für sukzessive Tests zum Einsatz gebracht werden. Hierbei wird die Aufwickelspule 22 durch den formschlüssig eingreifenden Antriebszapfen 24 um ihre Drehachse 25 rotierend angetrieben.

Die Geräteeinheit 10 weist ein langgestreckt-flaches Gehäuse 26 auf, das an seiner Vorderseite eine verschließbare Ladeöffnung 28 zum Wechseln der Kassette 14 besitzt. An einer Schmalseite des Gehäuses 26 ist eine Stechhilfe 30 angeflanscht, die es einem Benutzer ermöglicht, durch einen Hauteinstich eine Blutprobe zu gewinnen, die dann auf das Testfeld 18 aufgetragen werden kann. Eine nicht gezeigte Geräteelektronik ermöglicht die Messwertverarbeitung, wobei die geräteinterne Energieversorgung auch des Bandantriebs durch Batterien 32 erfolgt.

Wie auch aus Fig. 2 ersichtlich, weist der Bandantrieb 12 einen Elektromotor 34 und ein diesem nachgeordnetes Getriebe 36 auf. Der Elektromotor 34 ist als Gleichstrom-Kleinmotor ausgebildet, der mit hoher Drehzahl, beispielsweise bis zu 10.000 Umdrehungen in der Minute betrieben werden kann. Er weist einen langgestreckten Motorblock 38 auf, wobei die Motordrehachse 40 und entsprechend die Motorwelle 40' in Längsrichtung des Motorblocks 38 verläuft. Aufgrund des liegenden Einbaus des Motors 34 ist die Motordrehachse 40 parallel zu einer Breitseitenebene des Gehäuses 26 bzw. zur Öffnungsfläche der Ladeöffnung 28 orientiert, während die Spulenachse 25 senkrecht dazu verläuft. Um das axiale Spiel zu reduzieren, ist die Motorwelle 40' in Sinterlagern 42 angeordnet (in Fig. 2 nur symbolisch gezeigt). Zur weiteren Toleranzreduzierung ist der stirnseitig an dem Motorblock 38 fixierte Lagerbock 44 aus einem metallischen Werkstoff gefertigt.

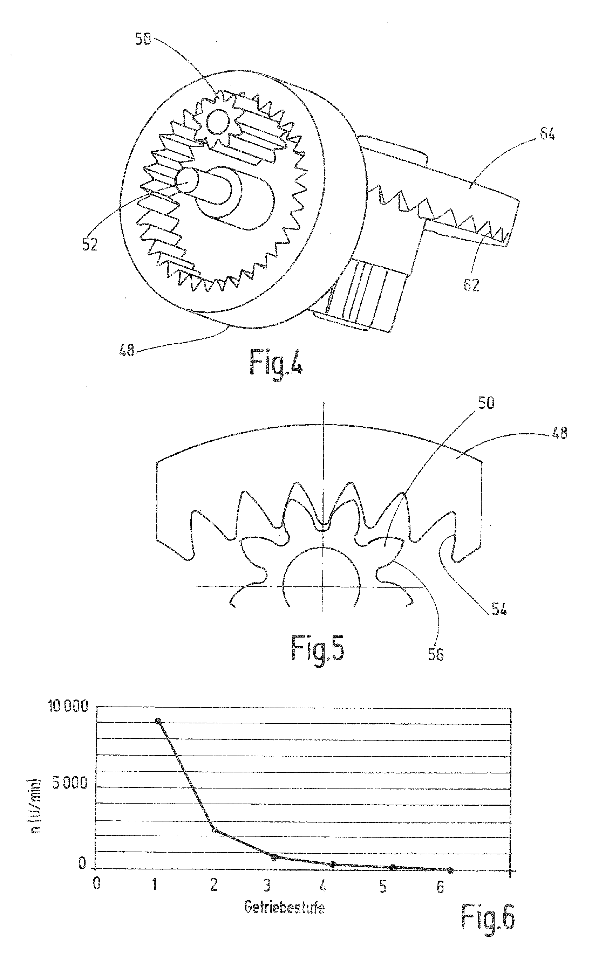

Die Übertragung der Antriebsbewegung zwischen Motor 34 und Aufwickelspule 22 erfolgt durch das Getriebe 36, welches zu diesem Zweck eine 90°-Umlenkung aufweist. Verschiedene Getriebestufen ermöglichen dabei die für das Vorspulen des Testbands 16 geeignete Drehzahl- und Drehmomentanpassung. Die funktionale Anordnung der Getriebeglieder ergibt sich auch aus der Seitenansicht gemäß Fig. 3 und der Detailvergrößerung nach Fig. 4.

Die mit der Motorwelle 40' direkt gekoppelte erste Getriebestufe ist als Hohlradgetriebestufe 46 ausgebildet. Diese umfasst ein innenverzahntes Hohlrad 48 und ein mit seiner Achse innerhalb des Hohlrads liegendes, auf der Motorwelle 40' sitzendes Antriebsritzel 50. Vorteilhafterweise ist das Hohlrad 48 über einen Lagerzapfen 52 an einer Lagerstelle des Lagerbocks 44 gelagert, so dass dort Positioniertoleranzen weitgehend minimiert sind und eine genaue Zentrierung der ersten Getriebestufe sichergestellt ist.

Um die Drehzahl und damit das Betriebsgeräusch schon im ersten Schritt erheblich zu reduzieren, weist die Hohlradgetriebestufe 46 ein hohes Übersetzungsverhältnis i = 4 auf. Zur Erzielung eines "weicheren" Geräusches weist die Innenverzahnung eine große Zahn-Überlappung auf. Dies lässt sich gemäß Fig. 5 dadurch realisieren, dass die Zähne des Hohlrads 48 mit leicht konkav gewölbten Zahnflanken 54 versehen sind, während das Antriebsritzel 50 konvex bzw. ballig geformte Zahnflanken 56 besitzt.

Die anschließende zweite Getriebestufe wird durch eine Kronenradgetriebestufe 58 gebildet, die für die 90°-Achsumlenkung in dem Antriebsstrang sorgt. Die Bewegungsübertragung erfolgt dabei durch ein koaxial mit dem Hohlrad 48 verbundenes Zahnrad 60, das mit der Kronenverzahnung 62 des Kronenrads 64 kämmt. Das Kronenrad 64 besitzt eine gegenüber dem Zahnrad 60 um 90° gedrehte Achse und ermöglicht zugleich eine große Untersetzung in einer Getriebestufe. Aufgrund der zylindrischen Form des Zahnrads 60 mit Geradverzahnung ist eine hohe axiale Verschiebetoleranz gewährleistet, ohne dass sich Probleme beim Laufverhalten ergeben. Ein geringes Geräusch und eine hohe Effizienz werden auch durch eine geringe Anzahl der Zähne und eine optimierte Zahngeometrie der Verzahnung 62 am Kronenrad 64 erzielt (s. auch Fig. 4).

Eine dritte Getriebestufe wird durch ein dem Kronenrad 64 nachgeordnetes mehrstufiges Stirnradgetriebe 66 gebildet. Dieses umfasst mehrere Stirnzahnrädern 68, 70 mit zueinander parallelen Drehachsen, die auf einer Seite einer gemeinsamen Lagerplatte 74 stabil gelagert sind (Fig. 2). Die Zahnräder bestehen zweckmäßig als Spritzgussteile aus POM (Polyoxymethylen). Das letzte Zahnrad 70 ist direkt mit einem als Metall-Stanzteil ausgebildeten gezahnten Spulenteller 72 verbunden, der an seiner freien Oberseite 76 den Antriebszapfen 24 für die Aufwickelspule 22 trägt.

Fig. 6 zeigt die Drehzahlreduzierung in der Reihenfolge der Getriebeglieder. Die Motordrehzahl n = 9.000 U/min wird durch die Hohlradgetriebestufe 46 schon im ersten Schritt stark reduziert, während die nachfolgenden Stufen eine eher gleichmäßige Reduzierung bewirken. In dem Diagramm ist Punkt 1 dem Antriebsritzel 50, Punkt 2 dem Hohlrad 48 und Punkt 3 dem Kronenrad 64 zugeordnet. Die Punkte 4, 5, 6 geben die Drehzahl der Zahnräder 68, 70, 72 an. An der Ausgangsseite wird somit eine Drehzahl des Antriebszapfens 24 und damit der Spule 22 von weniger als 25 Umdrehungen in der Minute erreicht, wobei auch das erforderliche Drehmoment zur Verfügung steht.

## Patentansprüche

1. Medizinisches Handgerät, insbesondere für Blutzuckertests, mit einer in ein Gerätegehäuse (26) einsetzbaren Testbandeinheit (14), die ein mit einer Vielzahl von analytischen Testelementen (18) versehenes, mittels einer Spule (22) aufwickelbares Testband (16) umfasst, und einem mit der Testbandeinheit (14) koppelbaren, einen Elektromotor (34) und ein Getriebe (36) umfassenden Bandantrieb (12) zum abschnittsweisen Vorspulen des Testbands (16), so dass die Testelemente (18) zur Probenapplikation sukzessive bereitstellbar sind, **dadurch gekennzeichnet, dass** die Drehachse des Elektromotors (34) in einer quer zur Drehachse der Spule (22) sich erstreckenden Ebene angeordnet ist, und dass das Getriebe (36) einen Winkeltrieb (58) zur Achsumlenkung aufweist.

2. Medizinisches Handgerät nach Anspruch 1, **dadurch gekennzeichnet, dass** der Winkeltrieb durch eine Kronenradgetriebestufe (58) gebildet ist.

3. Medizinisches Handgerät nach Anspruch 2, **dadurch gekennzeichnet, dass** die Kronenradgetriebestufe (58) ein Kronenrad (64) und ein zylindrisches Zahnrad (60) mit Geradverzahnung aufweist.

4. Medizinisches Handgerät nach einem der Ansprüche 1 bis 3, **dadurch gekennzeichnet, dass** das Getriebe (36) eine Hohlradgetriebestufe (46) aufweist, wobei die Hohlradgetriebestufe (46) durch ein innenverzahntes Hohlrad (48) und ein mit seiner Achse innerhalb des Hohlrads (48) liegendes, auf der Motorwelle (40') des Elektromotors (34) sitzendes Antriebsritzel (50) gebildet ist.

5. Medizinisches Handgerät nach Anspruch 4, **dadurch gekennzeichnet, dass** die Hohlradgetriebestufe (46) zur Drehzahlreduzierung ein Übersetzungsverhältnis von mehr als 2, vorzugsweise etwa 4 aufweist.

6. Medizinisches Handgerät nach Anspruch 4 oder 5,
**dadurch gekennzeichnet, dass** das Hohlrad (48) konkave Zahnflanken (54) und das Antriebsritzel (50) konvex gewölbte Zahnflanken (56) aufweist.

7. Medizinisches Handgerät nach einem der Ansprüche 4 bis 6, **dadurch gekennzeichnet, dass** die mit dem Elektromotor (34) direkt gekoppelte Hohlradgetriebestufe (46) an einer Lagerstelle an dem Elektromotor (34) gelagert ist.

8. Medizinisches Handgerät nach einem der Ansprüche 4 bis 7, **dadurch gekennzeichnet, dass** das Antriebsritzel (50) und ggf. die Lagerhalterung (44) für das Hohlrad (48) als metallische Bauteile ausgebildet sind.

9. Medizinisches Handgerät nach einem der Ansprüche 4 bis 8, **dadurch gekennzeichnet, dass** die Kronenradgetriebestufe (58) der Hohlradgetriebestufe (46) nachgeordnet ist, wobei die Motordrehzahl des Elektromotors (34) ein Mehrfaches der Eingangsdrehzahl der Kronenradgetriebestufe (58) beträgt.

10. Medizinisches Handgerät nach einem der Ansprüche 1 bis 9, **gekennzeichnet durch** eine mehrere Stirnzahnräder (68,70) umfassende, ausgangsseitig über einen Antriebszapfen (24) direkt mit der Spule (22) koppelbare Stirnradgetriebestufe (66).

11. Medizinisches Handgerät nach Anspruch 10, **dadurch gekennzeichnet, dass** die Stirnzahnräder (68,70) gemeinsam auf einer Seite einer Lagerplatte (74) gelagert sind.

12. Medizinisches Handgerät nach einem der Ansprüche 1 bis 11, **dadurch gekennzeichnet, dass** der Elektromotor (34) durch einen aus einer geräteseitigen Energieversorgung (32) gespeisten Gleichstrom-Kleinmotor mit einer Drehzahl im Bereich von 5000 bis 15000, vorzugsweise etwa 9000 Umdrehungen pro Minute gebildet ist.

13. Medizinisches Handgerät nach einem der Ansprüche 1 bis 12, **dadurch gekennzeichnet, dass** der Elektromotor (34) einen langgestreckten Motorblock (38) aufweist, und dass die Motorwelle (40') in Längsrichtung des Motorblocks (38) verläuft.

14. Medizinisches Handgerät nach einem der Ansprüche 1 bis 13, **dadurch gekennzeichnet, dass** die Motorwelle (40') des Elektromotors (34) in einem aus Sintermaterial bestehenden Sinterlager (42) gelagert ist.

15. Medizinisches Handgerät nach einem der Ansprüche 1 bis 14, **dadurch gekennzeichnet, dass** das Gerätegehäuse (26) zum Einwechseln einer Bandkassette als Testbandeinheit (14) ausgebildet ist, und dass die maximale Dicke des flachen Gerätegehäuses weniger als 30mm, vorzugsweise weniger als 25mm beträgt.
